# FASCICULE DE BREVET EUROPEEN

(11) **EP 2 097 293 B1**
(45) Date de publication et mention de la délivrance du brevet: **05.09.2012**
(21) Numéro de dépôt: 07858745.8
(22) Date de dépôt: 27.11.2007
(51) Int. Cl.: B60P 3/20

(54) **SYSTEME ET PROCEDE DE PREPARATION DUDIT SYSTEM DE CRYOTHERAPIE DU CORPS ENTIER**
SYSTEM UND VERFAHREN ZUR VORBEREITUNG DIESES SYSTEMS FÜR GANZKÖRPERKRYOTHERAPIE
SYSTEM AND METHOD OF PREPARING SAID SYSTEM FOR WHOLE-BODY CRYOTHERAPY

(30) Priorité: 07.12.2006 FR 0610658
(43) Date de publication de la demande: 09.09.2009
(73) Titulaire: Decourcelle, Olivier Marcel Maurice, 75006 Paris (FR); Barette, Gilles, 93370 Montfermeil (FR)
(72) Inventeur: Decourcelle, Olivier Marcel Maurice, 75006 Paris (FR); Barette, Gilles, 93370 Montfermeil (FR)
(74) Mandataire: Pontet, Bernard
(86) Numéro de dépôt international: PCT/FR2007/052407
(87) Numéro de publication internationale: WO 2008/068441

(56) Documents cités:
- WO-A-2006/045124
- US-A- 4 060 400
- US-A- 4 498 306
- US-A- 5 161 848
- US-A1- 2002 129 613
- US-B1- 6 378 319

## Description

La présente invention concerne un système, ainsi que son procédé de mise en place, pour la cryothérapie du corps entier applicable à des sujets humains ou animaux, ce système comprend une chambre de traitement ainsi que des moyens frigorifiques aptes à amener ou maintenir à une température inférieure à -80°C l'atmosphère de cette chambre de traitement. Selon l'invention, cette chambre de traitement et lesdits moyens frigorifiques, ou au moins l'un de ces deux éléments, sont montés sur au moins une structure autoporteuse mobile ou transportable, d'une façon permettant une mise en oeuvre depuis ladite structure autoporteuse.

Elle concerne en outre un système de cryothérapie du corps entier muni de préchambres à des températures intermédiaires, définissant un double parcours modifiable ou amovible entre la chambre de traitement et l'extérieur, et comprenant des moyens de cryothérapie locale.

Elle concerne également un tel système réparti en plusieurs modules séparables.

Le domaine principal de l'invention est celui des traitement par très grand froid, principalement appliqué aux êtres vivants, en particulier à des températures inférieures à -80°C, par exemple entre -100°C ou -120°C, souvent aux environs de -110°C.

Bien que dénommé usuellement "cryothérapie", un tel traitement peut avoir des visées autres que thérapeutiques, telles que de confort, d'esthétique ou de régularisation des performances physiques.

Depuis quelques années, des études ont démontré qu'une exposition du corps à des températures très froides, pendant une durée courte comprise entre 0,5 minute et quelques minutes, permet d'obtenir une réaction du corps produisant des effets sur une durée prolongée voire permanente.

De tels traitements sont basés sur une exposition directe de la peau à l'air à très basse température. Sur de courtes durées, la température intérieure n'est pas affectée et seule la température à la surface du corps baisse, jusqu'aux environs de +25°C.

Ainsi, des résultats ont été obtenus dans la prise en charge de certaines affections globales et chroniques dans les domaines de la traumatologie, rhumatologie et neurologie.

De tels types de traitement peuvent aussi servir à améliorer le confort général d'une personne en réactivant son métabolisme. Certains résultats peuvent aussi être obtenus sur le plan esthétique, par exemple par une action sur l'état ou l'aspect de la peau.

Un intérêt majeur est actuellement visible dans le domaine sportif, en particulier de compétition ou de haut niveau. Des études montrent que la récupération est rendue plus efficace et plus rapide après un effort intense, et les fragilisations ou risques de blessures sont diminués.

Sur une période prolongée, à l'entraînement mais aussi dans une compétition se prolongeant sur plusieurs jours ou semaines, les capacités physiques sont également régularisées.

Les systèmes existant actuellement sont installés dans certains centres spécialisés, de type thérapeutique ou dans certains complexes sportifs ou spécialisés en médecine du sport. Une telle installation est basée sur une chambre thermiquement isolée, refroidie par azote liquide ou par un compresseur spécial, par exemple avec trois étages de compression disposés en cascade.

De tels compresseurs relèvent d'une technologie très spécialisée, et diffèrent grandement des installations classiques de congélation, qui ne sont capables d'atteindre au mieux que -60°C environ, et le plus souvent -40°C seulement. Ce type de compresseur utilise des fluides spécifiques, représente une consommation importante, et représentent un coût important et n'ont été développés que récemment.

De plus, même ainsi, le temps de mise en température reste important, puisque le passage de -60°C à la température de traitement de - 110°C peut demander entre trois et quatre heures de fonctionnement.

De telles installations existent actuellement en peu d'exemplaires et sont d'un coût très important, ce qui ne permet pas d'envisager un équipement pour beaucoup de sites manquant de budget et/ou n'en ayant pas un usage suffisamment important.

Certains systèmes sont équipés d'une ou deux préchambres, dans lesquelles la température est maintenue à des températures intermédiaires, et par lesquelles transitent le ou les sujets pour accéder à la chambre de traitement ou en sortir.

De tels systèmes sont décrits dans le brevet européen EP 0 371 072, et sont proposés par des sociétés telle que Zimmer Elektromedizin ou Cryo Medizintechnik en Allemagne ou Xolod en Ukraine.

Dans certaines situations, la capacité d'un système peut parfois s'avérer insuffisante, même à deux préchambres, par exemple pour traiter dans le délai optimal la totalité des joueurs des deux équipes à l'issue d'une rencontre de sports collectifs, soit au moins 22 joueurs au football ou 30 au rugby. Ainsi, la capacité d'une installation de deux pièces est donnée pour 10-12 personnes/heure et jusqu'à 20-25 personnes/heure par les informations de la société Xolod.

Un but de l'invention est de pallier à tout ou partie de ces inconvénients, et en particulier:
- d'augmenter la souplesse d'utilisation d'un tel système;
- de faciliter l'accès à de tels traitements pour des utilisateurs ou des sites occasionnels ou ponctuels;
- d'améliorer les performances de ce type de traitement, en diminuant les délais entre l'effort physique et l'application du traitement;
- de diminuer les coûts d'accès à de tels traitements et de mieux utiliser les investissements consentis pour ce type de systèmes;
- de diminuer les temps de préparation d'un tel système;
- d'augmenter les capacités d'un tel système;
- de mieux intégrer ce type de traitement dans le cadre des différents types de physiothérapies existant, et d'optimiser leur combinaisons.

Dans ce but, l'invention propose un système de cryothérapie du corps entier pour sujets humains ou animaux, comprenant
au moins une chambre de traitement de dimensions suffisantes pour contenir au moins un sujet à traiter, ainsi que des moyens frigorifiques aptes à amener ou maintenir à une température inférieure à -80°C l'atmosphère de ladite chambre de traitement.
Selon l'invention, ladite chambre de traitement et lesdits moyens frigorifiques, ou au moins l'un de ces deux éléments, sont montés sur au moins une structure autoporteuse mobile ou transportable, d'une façon permettant une mise en oeuvre depuis ladite structure autoporteuse. Cette structure autoporteuse peut être par exemple un module ou un conteneur contenant ces éléments ou une plateforme supportant ces éléments.

Plus particulièrement, la structure autoporteuse est intégrée à un véhicule routier ou une remorque routière, ou comporte des moyens de fixation à un tel véhicule ou remorque. Cette structure autoporteuse peut aussi comporter des moyens de préhension permettant la manutention de ladite structure autoporteuse par des moyens de levage ou de manutention, qui peuvent être eux-mêmes fixés sur un tel véhicule ou remorque.

Il est ainsi possible de déplacer le système de cryothérapie, pour pouvoir le mettre en place de façon provisoire et ponctuelle sur le lieu où il est demandé. Ce système peut être déplacé en une ou plusieurs parties, par exemple dans des modules ou des conteneurs monoblocs, pouvant être fixés ou juxtaposés entre eux. Les modules constituent des cellules assemblables et comportant les connexions nécessaires pour rendre l'ensemble opérationnel de façon simple tout en évitant ou limitant les opérations de montage ou de réglage à la mise en route.

Il devient ainsi possible de proposer un service mobile à la demande, en tout lieu, moyennant un préavis de quelques heures à quelques jours.

Ce type de service peut s'adresser à de nombreux utilisateurs qui n'avaient pas jusqu'à présent le besoin ou les moyens d'investir dans une installation sur place, par exemple de petites formations sportives pour quelques occasions particulières, à un coût correspondant à une telle utilisation.

De plus, il est ainsi possible de pratiquer un tel traitement immédiatement à l'issue d'un effort, par exemple à la sortie d'une rencontre sportive, même si la rencontre se déroule sur un lieu non équipé. En effet, l'effet de récupération et de lissage des performances est meilleur si le traitement est appliqué dans un délai relativement court après l'effort, qui ne permet pas toujours d'envisager un déplacement du ou des sujets vers un lieu équipé.

En s'adressant une multiplicité de clients occasionnels ou ponctuels, il est aussi plus facile d'amortir l'investissement que représente un tel système, malgré l'éventuel surcoût que peut engendrer à la construction la nature mobile dudit système.

Avantageusement, la chambre de traitement communique avec l'extérieur par l'intermédiaire d'une ou plusieurs préchambres successives, chacune de dimensions suffisantes pour contenir au moins un sujet à traiter, et dont les atmosphères respectives sont amenées ou maintenues à une ou plusieurs températures intermédiaires entre la température extérieure et la température de la chambre de traitement.

Selon l'inventioh, le système peut comprendre des moyens de cryothérapie locale utilisables depuis l'intérieur d'au moins une préchambre. En appliquant une telle thérapie locale à la sortie de la chambre de traitement, ses effets sont alors potentialisés par le passage préalable à - 110°C.

La température de la peau d'un sujet humain dans un environnement à +20°C est normalement d'environ +32°C. La cryothérapie locale consiste à refroidir la peau d'au moins 15°C pour enclencher une réaction de vasodilatation depuis l'intérieur du corps. Après l'exposition à -110°C, la température superficielle est déjà descendue, par exemple aux environs de +25°C. Une cryothérapie locale est appliquée dans une préchambre à -60°C ou à -10°C pour amener la peau à la température déclenchant la vasodilatation, par exemple autour de +17°C. Les effets de cette cryothérapie locale seront plus efficaces du fait de l'ambiance froide et de la réaction déjà enclenchée dans le corps du sujet, et seront également obtenus plus rapidement du fait de la température superficielle déjà abaissée. A titre d'exemple, un tel traitement local qui prendrait environ 10 mn dans un environnement à +20°C peut ne nécessiter qu'environ une minute dans un environnement à -10°C et après exposition à -110°C.

Avantageusement, au moins une préchambre peut comprendre un dispositif de séparation physique définissant au moins deux parcours séparés entre
- d'une part au moins une ouverture communiquant avec l'espace adjacent de température inférieure à la température de ladite préchambre, et
- d'autre part au moins une ouverture communiquant avec l'espace adjacent de température supérieure à la température de ladite préchambre.

Il est ainsi possible d'utiliser l'un de ces parcours comme parcours d'entrée dans la chambre de traitement et l'autre comme parcours de sortie. Une telle séparation physique rendra l'organisation du flux des sujets traités plus naturelle et plus efficace. En séparant les sujets entrant et sortant dans l'une des préchambres ou dans les deux, il est aussi possible d'augmenter la capacité globale du système en faisant rentrer de nouveaux sujets dans chaque préchambre en même temps que d'autres en sortent, sans risque de confondre les uns et les autres, et en limitant les risques de bousculades et de contact avec les parois. Il est ainsi possible d'utiliser la chambre de traitement plus près de son maximum de capacité théorique. La capacité obtenue peut alors dépasser 30 ou 40 personnes/heure pour une exposition de 2 minutes dans une chambre de 5 personnes.

Dans certains modes de réalisation de l'invention, le système comprend au moins deux modules séparables dont l'un au moins est monté sur une structure autoporteuse mobile ou transportable, de façon à permettre une mise en oeuvre depuis ladite structure autoporteuse, l'un desdits modules comprenant au moins la chambre de traitement, et l'autre desdits modules comprenant au moins les moyens frigorifiques ou au moins une préchambre.

De tels modules peuvent être transportés sur des véhicules différents, ou répartis sur un véhicule et sa remorque, ce qui rend le système moins encombrant et d'un transport plus souple.

Ainsi, il est aussi possible de déplacer séparément les différents éléments, selon les besoins.

Dans un de ces modes de réalisation, les moyens frigorifiques sont montés sur une structure autoporteuse mobile ou transportable. Le système comprend alors des moyens de connexion pour relier lesdits moyens frigorifiques de façon amovible à une ou plusieurs chambres de traitement construites ou transportées indépendamment desdits moyens frigorifiques.

Une telle configuration permet de positionner à l'avance la chambre de traitement et gagner du temps sur l'installation. Elle permet aussi d'installer plusieurs chambres de traitements, possiblement permanentes, dans différents endroits. Ces différentes chambres de traitement peuvent alors être utilisées avec un même module frigorifique apporté au moment voulu, et dont le coût sera ainsi plus facilement amorti ou rentabilisé.

Dans un autre de ces modes de réalisation, les moyens frigorifiques et la chambre de traitement sont montés sur une ou plusieurs structures autoporteuses mobiles ou transportables, et le système peut comprendre des moyens de connexion pour relier ladite chambre de traitement de façon amovible à une ou plusieurs préchambres construites ou transportées indépendamment desdits moyens frigorifiques.

Les préchambres peuvent ainsi faire partie d'installations fixes, telles que des vestiaires ou un centre de physiothérapie. La cryothérapie peut ainsi être pratiquée dans un lieu où sont réunis des moyens et du personnel nécessaires à d'autres traitements, complémentaires ou utiles aux mêmes sujets. Ces autres traitements peuvent en particulier comprendre électrothérapie, luminothérapie, laser, massages, hydrothérapie, et d'autres techniques de physiothérapie.

Dans des modes de réalisation pouvant être combinés avec tout ou partie des précédents, le système comprend en outre des deuxièmes moyens frigorifiques, par exemple un groupe de réfrigération d'une technologie plus classique et moins coûteuse.

Ces deuxièmes moyens peuvent assurer le fonctionnement de moyens de cryothérapie locale. Ils peuvent aussi assurer tout ou partie de l'obtention ou du maintien en température d'au moins une préchambre d'une température supérieure à la chambre de traitement. Ils peuvent encore assurer un complément ou une préparation à l'obtention ou au maintien en température de la chambre de traitement.

Pour ces besoins, les performances maximales des moyens frigorifiques principaux ne sont pas toujours nécessaires. De tels moyens secondaires sont alors plus économiques et plus simples à faire fonctionner, peuvent être plus fiables et plus solides, nécessiter moins de maintenance, et mieux à même de fonctionner au cours du transport.

Ainsi, l'invention propose également un procédé de préparation d'un tel système de cryothérapie du corps entier.

Ce procédé comprend une étape de transport des moyens frigorifiques et/ou de la chambre de traitement sur un site choisi pour une utilisation ponctuelle ou non permanente, suivie d'une étape de mise en température de la chambre de traitement.

D'autres particularités et avantages de l'invention ressortiront de la description détaillée d'un mode de mise en oeuvre nullement limitatif, et des dessins annexés sur lesquels :
- la FIGURE 1 est un schéma en vue latérale illustrant un système selon l'invention monté sur semi-remorque;
- la FIGURE 2 est un schéma en vue de dessus illustrant l'organisation d'un système selon l'invention monté sur un véhicule poids lourd;
- la FIGURE 3 est un schéma en vue de dessus illustrant l'organisation d'un système selon l'invention dans un mode de réalisation à géométrie variable;
- la FIGURE 4 est un schéma en vue latérale illustrant un système selon l'invention monté sur véhicule porteur et remorque séparée;
- la FIGURE 5 est un schéma en vue de dessus illustrant un mode de réalisation de l'invention avec moyens frigorifiques mobiles chambre de traitement et préchambres fixes;
- la FIGURE 6 est un schéma en vue de dessus illustrant un mode de réalisation de l'invention avec moyens frigorifiques mobiles et chambre de traitement fixe.

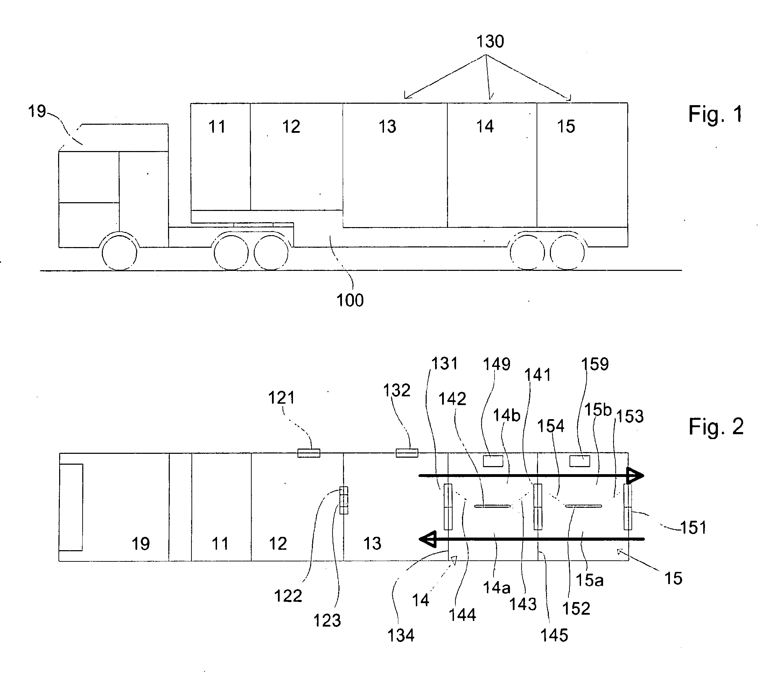

Les FIGURE 1 et FIGURE 2 illustre un mode de réalisation de l'invention où l'ensemble des éléments du système de cryothérapie sont montés de façon permanente et fonctionnelle sur véhicule poids lourd, ici sous la forme d'une semi-remorque 100 constituant la structure autoporteuse et adaptable à un tracteur routier 19. Le principe du véhicule semi-remorque permet une certaine souplesse d'utilisation et une économie d'investissement du fait du tracteur routier séparable et interchangeable. Un mode de réalisation proche pourrait aussi être réalisé sur la base d'un véhicule de type autocar, pour une meilleure compacité sur la route et une présentation plus typée tourisme et confort.

Les parties embarquées comprennent, en partant de l'avant de partie fonctionnelle : un compartiment technique 11 réunissant le compresseur frigorifique spécifique et un groupe électrogène, la salle de contrôle 12, puis la chambre de très grand froid 130.

Les éléments frigorifiques et de contrôle peuvent typiquement être de même type que dans les installations fixes existantes, telles que fournie par exemple par la société Cryo Medizin et/ou la société Bock.

Le compartiment technique 11 comprend un groupe électrogène alimentant tout ou partie du système, en particulier en route. Il comprend aussi les moyens frigorifiques, par exemple un compresseur spécifique triple étage permettant d'obtenir la température de -110°C dans la chambre de traitement.

Les moyens frigorifiques sont suspendus par au moins une structure suspendue, montée de façon à permettre une mise en oeuvre depuis ladite structure suspendue, limitant ainsi les vibrations qu'ils reçoivent lors du transport.

Une telle structure suspendue est par exemple un châssis supportant l'ensemble des éléments du compresseur triple étage, et monté sur la structure autoporteuse 100 par l'intermédiaire de moyens d'amortissement d'un type connu, tels qu'un matériau déformable de type mousse haute densité, ou une mousse à cellules fermées, ou des silentblocs en caoutchouc, un système à ressorts plus amortisseurs hydrauliques ou à suspension hydraulique ou oléopneumatique.

Dans le cas d'un système avec modules séparables ou déchargeables en caissons tel que décrit plus loin, les moyens de suspension peuvent aussi être situés à l'extérieur de la structure autoporteuse, c'est à dire par exemple entre le véhicule et le caisson.

La salle de contrôle 12 comprend tous les moyens de commande et de suivi du fonctionnement du système et de suivi du traitement. Elle dotée d'une porte d'accès extérieure 121 et d'une porte 122 thermiquement isolée communiquant avec la chambre de traitement 13, et munie d'une fenêtre 123 permettant une vue sur l'intérieur de la chambre de traitement 13.

La chambre de très grand froid est ici d'un type appelé "tri-sas" et comportant trois cabines 13, 14 et 15 successives, thermiquement isolées et communiquant entre elles par des ouvertures munies de portes thermiquement isolées.

Certains des modes de réalisation décrits ici peuvent cependant parfaitement être combinées avec une architecture de type "bi-sas" ou "mono-sas".

L'entrée depuis l'extérieur se fait par la cabine 15, par une porte extérieure 151, donnant sur une plateforme ou munie d'une échelle d'accès lors de l'utilisation. Cette cabine 15 constitue une première préchambre et forme un palier intermédiaire en température, avec une température intérieure de -10°C lors du fonctionnement.

Depuis cette première préchambre 15 on peut accéder par une porte 141 à une autre cabine constituant une deuxième préchambre 14, où règne une température intérieure de -60°C. Depuis cette deuxième préchambre 14, on peut accéder à une autre cabine constituant la chambre de traitement 13. Cette chambre de traitement 13 est aussi munie d'une porte extérieure 132, pouvant servir d'accès technique ou de porte de secours.

Ainsi que l'on voit en FIGURE 2, la première préchambre 15 et la deuxième préchambre 14 sont subdivisées chacune en deux parties 15a, 15b et respectivement 14a, 14b par un séparateur physique, par exemple une cloison 152, 142 ou possiblement une simple rambarde.

Dans la première préchambre 15, cette cloison 152 va de la porte extérieure 151 à une porte 141 communiquant avec la deuxième préchambre 14. Dans la deuxième préchambre 14, cette cloison 142 va de cette même porte d'accès 141 à la porte 131 communiquant avec la chambre de traitement 13.

Cette cloison 152 est munie à chacune de ses extrémités d'un portillon mobile 153 et 154, qui peut être commandé de la salle de contrôle 12. Ces portillons 153 et 154 peuvent aussi être conçus pour se déplacer automatiquement ou être déplacé manuellement de façon à ne permettre le passage vers chacune des parties 15a respectivement 15b de la préchambre 15 qu'en provenance de l'une des portes d'accès 151 respectivement 141.

De la même façon, la cloison 142 de la deuxième préchambre 14 est munie à chacune de ses extrémités d'un portillon mobile 143 et 144, qui peut être commandé de la salle de contrôle 12. Ces portillons 143 et 144 peuvent aussi être conçus pour se déplacer automatiquement ou être déplacé manuellement de façon à ne permettre le passage vers chacune des parties 14a respectivement 14b de la préchambre 14 qu'en provenance de l'une des portes d'accès 141 respectivement 131.

Cette subdivision par les cloisons 152 et 142, ainsi que le fonctionnement de des portillons correspondants, permet d'organiser une circulation dans un seul sens entre l'extérieur et la chambre de traitement par les premières parties 15a et 14a des deux préchambres dans le sens de l'entrée, et par les deuxièmes parties 14b et 15b de ces mêmes préchambres dans le sens de la sortie.

Dans la première préchambre 15 et la deuxième préchambre 1, ou seulement l'une des deux, de préférence dans la partie 14b, 15b servant au parcours de sortie, des moyens de traitement par cryothérapie locale 149 et 159 sont prévus, par exemple d'un type connu avec une buse mobile projetant de l'air froid entre -20°C et -45°C. Ces moyens peuvent ainsi être utilisés pour le traitement spécifique de certaines parties du corps telles que les articulations où des affections musculaires localisées, avec des effets renforcés et facilités par la combinaison avec le traitement du corps entier.

Dans une variante non représentée, ces différentes portes 151, 141 et 131 sont suffisamment larges, ou s'ouvrent en deux parties, ou comprennent chacune deux portes séparées, pour permettre la circulation dans les deux sens simultanément sans portillon à manoeuvrer.

De plus, une ou plusieurs cloisons réalisant au moins un dispositif de séparation physique entre deux préchambres ou au sein d'une préchambre, sont mobiles ou amovibles.

Il est ainsi possible de passer d'une configuration à une autre facilement selon les besoins, par exemple en retirant des cloisons ou des séparateurs pour agrandir l'espace disponible et traiter des sujets particulièrement encombrants, tels que des chevaux de course.

Une configuration possible consiste alors à retirer les cloisons 142 et 152 de chacune des préchambres 14 et 15, pour augmenter la largeur disponible ou faciliter l'entretien.

La séparation entre la première préchambre 15 et la deuxième préchambre 14 peut aussi être prévue mobile ou amovible, ce qui permet d'obtenir une seule préchambre de plus grande longueur. En retirant la cloison 145 entre la première préchambre 15 et la deuxième préchambre 14, on obtient ainsi un système de type "bi-sas".

La porte extérieure 151 et la porte 131 d'accès à la chambre de traitement sont prévues d'une dimension suffisante pour laisser passer de tels sujets encombrants, par exemple par deux battants indépendants.

La séparation entre la deuxième préchambre 14 et la chambre de traitement 13 peut aussi être prévue mobile ou amovible. En retirant la cloison 134 entre la deuxième préchambre 14 et la chambre de traitement 13, on obtient ainsi un système de type "mono-sas" d'une capacité plus importante.

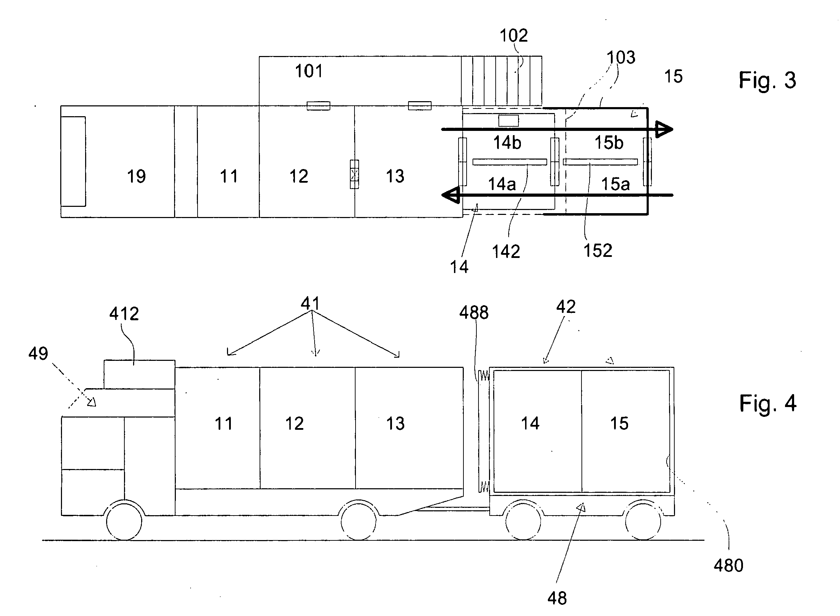

En FIGURE 3 est illustré un mode de réalisation où le système comporte au moins une partie mobile 101, 103 permettant une modification des dimensions horizontales d'au moins un élément qu'il supporte. Une telle configuration à géométrie variable, par exemple dépliable ou extensible, permet de disposer d'un espace suffisant à l'arrêt et en utilisation, sans générer un encombrement trop important lors du transport ni dépasser certains gabarits réglementaires ou de passage sur la route.

Dans cet exemple, la semi-remorque 100 comprend une plateforme latérale 101 pouvant coulisser sous le plancher des cabines 12 et 13 ou pouvant se rabattre sur l'extérieur de leur paroi latérale. Cette plateforme 101 est munie d'un escalier dépliable 102 et permet, à l'arrêt, un accès pratique et sûr à la salle de contrôle 21 et à la porte extérieure de la chambre de traitement 13.

Dans sa partie arrière, la semi-remorque 100 comprend une structure 103 s'emboîtant autour des parois extérieures de la deuxième préchambre 14, et pouvant coulisser vers l'arrière pour libérer un espace constituant la première préchambre 15, dans laquelle peuvent alors être montées les cloisons amovibles 152 et 142.

Une telle géométrie variable peut bien sûr être combinée avec d'autres modes de réalisation de l'invention, par exemple avec un véhicule unique, ou un caisson déchargeable ou une remorque indépendante.

En FIGURE 4 est illustré un mode de réalisation de l'invention où le système est réparti en deux modules séparés pouvant être connectés entre eux pour le fonctionnement.

Un premier module 41 est porté par un véhicule poids lourd 49 et un deuxième module 42 est porté par une remorque 48, qui peut être tractée par ce même véhicule 49 ou être déplacée indépendamment.

Dans cet exemple, le véhicule principal 49 porte le compartiment technique 11, la salle de contrôle 12 et la chambre de traitement 13, et la remorque 48 porte les deux préchambres 14 et 15. L'un ou l'autre des deux modules 41 et 42 comporte en outre des moyens de connexion 488 permettant une connexion simple et réversible des différents fluides ou informations, ainsi qu'une protection et une isolation thermique du passage entre différentes zones 13, 14 de basse température.

Pendant l'étape de transport, le procédé peut aussi inclure un pré-refroidissement d'au moins la chambre de traitement par des deuxièmes moyens frigorifiques aptes à fonctionner pendant le déplacement ou les étapes dudit déplacement.

Ce pré-refroidissement est assuré par des deuxièmes moyens frigorifiques 412 capable de fonctionner en route ou par raccordement au secteur électrique aux étapes, par exemple un groupe classique à compresseur tel qu'utilisé dans les transports de surgelé.

Le temps de mise en température complet après arrivée ou installation sur place sera ainsi diminué.

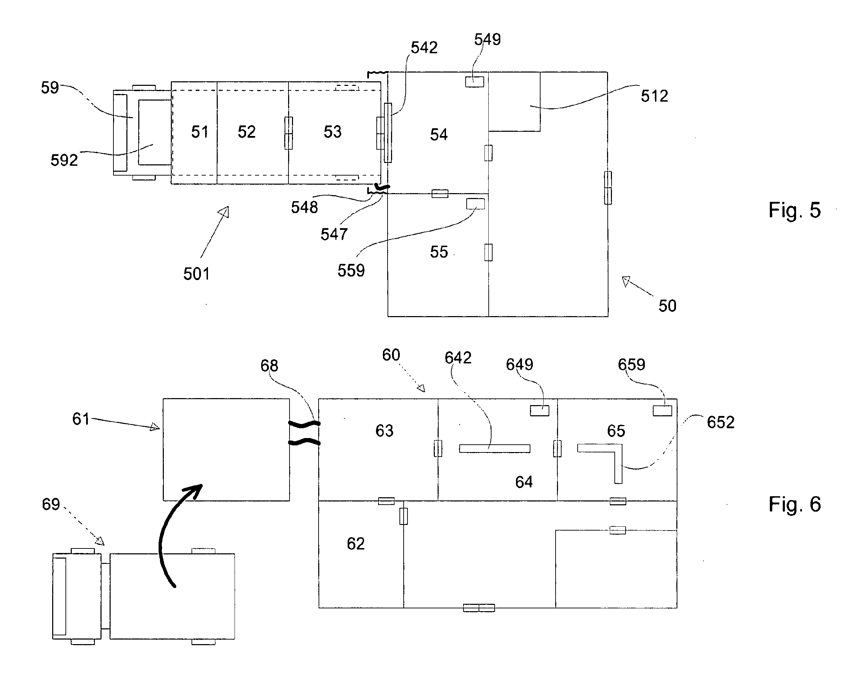

En FIGURE 5 est illustré une mise en oeuvre du système dans un mode de réalisation comprenant la chambre de traitement montée sans préchambres sur un véhicule 59.

Le véhicule embarque un compartiment technique 51, la salle de contrôle 52 et une chambre de traitement 53. Ce véhicule vient se placer devant une préchambre 54 faisant partie d'une installation fixe, par exemple au sein d'un bâtiment 50. Le système comprend des moyens de connexion, par exemple une liaison électrique et informatique 547 et un sas extensible 548, qui peut être monté à l'extérieur de la préchambre 54 ou à l'arrière de la chambre de traitement 53.

Selon l'invention, l'étape de transport est alors suivie d'une étape pour connecter ou assujettir la chambre de traitement à au moins une préchambre construite ou transportée indépendamment de la chambre de traitement, permettant ainsi d'utiliser des installations existant sur place ou apportées préalablement.

Le système comprend aussi des deuxièmes moyens frigorifiques embarqués 592, capables de fonctionner en cours de route ou aux étapes, pour assurer un pré-refroidissement de la chambre de traitement 53, et ainsi diminuer le temps de préparation de l'ensemble après arrivée du véhicule.

L'installation 50 comprend aussi un local technique 512 comprenant des deuxièmes moyens frigorifiques fixes 512, qui permettent d'assurer le refroidissement ou le pré-refroidissement de la deuxième préchambre 54, ainsi que le refroidissement de la première préchambre 55 et le fonctionnement des dispositifs de traitement local 549 et 559 des première et deuxième préchambres 55 et 54.

En FIGURE 6 est illustré un mode de réalisation de l'invention où le système comprend un module 61 incluant les moyens frigorifiques spécifiques. Ce module est monté sur un véhicule 69, par exemple dans un caisson autoporteur déchargeable à l'aide d'une grue embarquée ou non.

Ce module est amené à proximité d'une installation, pouvant elle-même être fixe ou mobile en tout ou partie, par exemple intégrée à un bâtiment 60. Cette installation comprend une chambre de traitement 63 et une salle de contrôle 62 auxquelles les moyens frigorifiques du module 61 sont connectés par des moyens de connexion 68 de fluides et d'informations. Les moyens frigorifiques du module 61 peuvent aussi comporter une partie mobile, par exemple comprenant un groupe condenseur, en permanence connectée au reste dudit module, et qui peut être déplacé sur une courte distance pour s'accoupler ou s'insérer dans ou auprès de la chambre de traitement 13.

L'installation 60 comprend aussi un local technique 612 comprenant des deuxièmes moyens frigorifiques, qui permettent d'assurer le pré-refroidissement de la chambre de traitement 63 et de la deuxième préchambre 64, ainsi que le refroidissement de la première préchambre 65 et le fonctionnement des dispositifs de traitement local 649 et 659 des première et deuxième préchambres 65 et 64.

Dans les différents modes de réalisation de l'invention, chaque module peut aussi, en variante, soit être intégré à un véhicule ou remorque soit être monté sur un châssis ou dans un caisson 480 séparable dudit véhicule ou remorque 48.

Bien sûr, l'invention n'est pas limitée aux exemples qui viennent d'être décrits et de nombreux aménagements peuvent être apportés à ces exemples sans sortir du cadre de l'invention.

## Revendications

1. Système de cryothérapie du corps entier pour sujets humains ou animaux, comprenant au moins une chambre de traitement (13, 53, 63) de dimensions suffisantes pour contenir au moins un sujet à traiter, ainsi que des moyens frigorifiques (11, 51, 61) par compresseur aptes à amener ou maintenir à une température inférieure à -80°C l'atmosphère de ladite chambre de traitement, **caractérisé en ce que** ladite chambre de traitement et lesdits moyens frigorifiques, ou au moins l'un de ces deux éléments, sont montés sur au moins une structure autoporteuse mobile (100, 41, 59, 501) ou transportable (61), et sont agencés sur ladite structure autoporteuse de façon à permettre une mise en oeuvre depuis ladite structure autoporteuse.

2. Système selon la revendication précédente, **caractérisé en ce que** la structure autoporteuse (100, 41, 501, 61) est intégrée à un véhicule routier (19, 59) ou une remorque routière (100), ou comporte des moyens de fixation à un tel véhicule (69) ou remorque, ou des moyens de préhension permettant la manutention de ladite structure par des moyens de levage ou de manutention.

3. Système selon l'une des revendications précédentes, **caractérisé en ce que** les moyens frigorifiques ( 11, 51, 61) sont suspendus par au moins une structure suspendue, montée de façon à permettre une mise en oeuvre depuis ladite structure suspendue.

4. Système selon l'une des revendications précédentes, **caractérisé en ce que** la chambre de traitement (13, 53, 63) communique avec l'extérieur par l'intermédiaire d'une ou plusieurs préchambres (14, 15, 54, 55, 64, 65) successives, chacune de dimensions suffisantes pour contenir au moins un sujet à traiter, et dont les atmosphères respectives sont amenées ou maintenues à une ou plusieurs températures intermédiaires entre la température extérieure et la température de la chambre de traitement.

5. Système selon la revendication 4, **caractérisé en ce qu'**il comprend des moyens de cryothérapie locale utilisables depuis l'intérieur d'au moins une préchambre.

6. Système selon l'une des revendications 4 à 5, **caractérisé en ce qu'**au moins une préchambre (14, 15, 54, 55, 64, 65) comprend un dispositif de séparation physique (142, 152, 642, 652) définissant au moins deux parcours séparés entre d'une part au moins une ouverture (131) communiquant avec l'espace (13) adjacent de température inférieure à la température de ladite préchambre (14), et d'autre part au moins une ouverture (141) communiquant avec l'espace (15) adjacent de température supérieure à la température de ladite préchambre.

7. Système selon l'une des revendications 4 à 6, **caractérisé en ce qu'**il comprend une ou plusieurs cloisons mobiles ou amovibles réalisant au moins un dispositif de séparation physique (145) entre deux préchambres (14 et 15) ou au sein (142, 152) d'une préchambre (14, 15).

8. Système selon l'une des revendications précédentes, **caractérisé en ce qu'**il comprend au moins deux modules séparables dont l'un au moins est monté sur une structure autoporteuse mobile (48 ; 59) ou transportable (61), et est agencé sur ladite structure autoporteuse de façon à permettre une mise en oeuvre depuis ladite structure autoporteuse, l'un (41 ; 501 ; 60) desdits modules comprenant au moins la chambre de traitement (13 ; 53 ; 63), et l'autre (42 ; 50 ; 61) desdits modules comprenant au moins les moyens frigorifiques (61) ou au moins une préchambre (14, 15 ; 54, 55).

9. Système selon l'une des revendications 2 à 8, **caractérisé en ce que** le véhicule routier ou la remorque routière (100) comporte au moins une partie mobile (101, 103) permettant une modification des dimensions horizontales d'au moins un élément (12, 13, 14, 15) qu'il supporte.

10. Système selon l'une des revendications 8 à 9, **caractérisé en ce que** les moyens frigorifiques sont montés sur une structure autoporteuse mobile ou transportable (61), ledit système comprenant des moyens de connexion (68) pour relier lesdits moyens frigorifiques (61) de façon amovible à une ou plusieurs chambres de traitement (63) construites ou transportées indépendamment desdits moyens frigorifiques.

11. Système selon l'une des revendications 8 à 10, **caractérisé en ce que** les moyens (51) frigorifiques et la chambre de traitement (53) sont montés sur une ou plusieurs structures autoporteuses mobiles (59, 501) ou transportables, ledit système comprenant des moyens de connexion (547, 548) pour relier ladite chambre de traitement (53) de façon amovible à une ou plusieurs préchambres (54, 55) construites ou transportées indépendamment desdits moyens frigorifiques.

12. Système selon l'une des revendications précédentes, **caractérisé en ce qu'**il comprend en outre des deuxièmes moyens frigorifiques (412, 512, 592, 612) assurant le fonctionnement de moyens de cryothérapie locale (149, 159, 549, 559, 649, 659) ; ou assurant tout ou partie de l'obtention ou du maintien en température d'au moins une préchambre (14, 15, 54, 55, 64, 65) d'une température supérieure à la chambre de traitement; assurant un complément ou une préparation à l'obtention ou au maintien en température de la chambre de traitement (13, 53, 63).

13. Procédé de préparation d'un système selon l'une des revendications précédentes, **caractérisé en ce qu'**il comprend une étape de transport des moyens frigorifiques (11, 51, 61) et de la chambre de traitement (13, 53), ou au moins des moyens frigorifiques, sur un site (50, 60) choisi pour une utilisation ponctuelle ou non permanente, ladite étape de transport étant au moins suivie d'une étape de mise en température de la chambre de traitement jusqu'à sa température de fonctionnement.

14. Procédé selon la revendication 13, **caractérisé en ce que** l'étape de transport inclut un pré-refroidissement d'au moins la chambre de traitement (13, 53) par des deuxièmes moyens frigorifiques (412, 512) aptes à fonctionner pendant le déplacement ou les étapes dudit déplacement.

15. Procédé selon l'une des revendications 13 à 14, **caractérisé en ce que** l'étape de transport est suivie d'une étape pour connecter ou assujettir la chambre de traitement (53) à au moins une préchambre construite (54) ou transportée indépendamment de la chambre de traitement.

## Claims

1. Whole-body cryotherapy system for human or animal subjects, comprising at least one treatment chamber (13, 53, 63) of sufficient dimensions to contain at least one subject to be treated, as well as refrigeration means (11, 51, 61) by compressor capable of bringing the atmosphere of said treatment chamber to a temperature below -80°C or maintaining it at that temperature, **characterized in that** said treatment chamber and said refrigeration means, or at least one of these two elements, are mounted on at least one self-supporting mobile (100, 41, 59, 501) or transportable (61) structure and are arranged on said self-supporting structure so as to allow operation from said self-supporting structure.

2. System according to the previous claim, **characterized in that** the self-supporting structure (100, 41, 501, 61) is integrated with a road vehicle (19, 59) or trailer (100), or comprises means of attachment to such a vehicle (69) or trailer, or gripping means allowing the handling of said structure by lifting or handling means.

3. System according to one of the previous claims, **characterized in that** the refrigeration means (11, 51, 61) are suspended by at least one suspended structure, mounted so as to allow operation from said suspended structure.

4. System according to one of the previous claims, **characterized in that** the treatment chamber (13, 53, 63) communicates with the outside by means of one or more successive antechambers (14, 15, 54, 55, 64, 65), each having dimensions sufficient to contain at least one subject to be treated, and the respective atmospheres of which are brought to, or maintained at, one or more intermediate temperatures between the outside temperature and the temperature of the treatment chamber.

5. System according to claim 4, **characterized in that** it comprises local cryotherapy means which can be used from within at least one antechamber.

6. System according to one of claims 4 to 5, **characterized in that** at least one antechamber (14, 15, 54, 55, 64, 65) comprises a physical separation device (142, 152, 642, 652) defining at least two separate routes between, on the one hand, at least one opening (131) communicating with the adjacent space (13) having a temperature below the temperature of said antechamber(14), and on the other hand, at least one opening (141) communicating with the adjacent space (15) having a temperature above the temperature of said antechamber.

7. System according to one of claims 4 to 6, **characterized in that** it comprises one or more moveable or detachable partitions providing at least one physical separation device (145) between two antechambers (14 and 15) or within (142, 152) one antechamber (14, 15).

8. System according to one of the previous claims, **characterized in that** it comprises at least two separable modules at least one of which is mounted on a mobile (48 ; 59) or transportable (61) self-supporting structure, in order to allow operation from said self-supporting structure, one (41 ; 501 ; 60) of said modules comprising at least the treatment chamber (13 ; 53 ; 63), and the other (42 ; 50 ; 61) of said modules comprising at least the refrigeration means (61) or at least one antechamber(14, 15 ; 54, 55).

9. System according to one of claims 2 to 8, **characterized in that** the road vehicle or the road trailer (100) comprises at least one moveable part (101, 103) allowing a modification of the horizontal dimensions of at least one element (12, 13, 14, 15) that it supports.

10. System according to one of claims 8 to 9, **characterized in that** the refrigeration means are mounted on a mobile or transportable self-supporting (61) structure, said system comprising connection means (68) for connecting said refrigeration means (61) detachably to one or more treatment chambers (63) constructed or transported independently of said refrigeration means.

11. System according to one of claims 8 to 10, **characterized in that** the refrigeration means (51) and the treatment chamber (53) are mounted on one or more mobile (59, 501) or transportable self-supporting structures, said system comprising connection means (547, 548) for connecting said treatment chamber (53) detachably to one or more treatment chambers (54, 55) constructed or transported independently of said refrigeration means.

12. System according to one of the previous claims, **characterized in that** it comprises moreover second refrigeration means (412, 512, 592, 612) ensuring operation of the local cryotherapy means (149, 159, 549, 559, 649, 659); or ensuring all or part of bringing the temperature of at least one antechamber (14, 15, 54, 55, 64, 65) to a temperature above that of the treatment chamber or maintaining it at that temperature; providing additional means for reaching or maintaining the temperature of the treatment chamber (13, 53, 63) or preparation therefor.

13. Method for the preparation of a system according to one of the previous claims, **characterized in that** it comprises a stage of transporting the refrigeration means (11, 51, 61) and the treatment chamber (13, 53), or at least the refrigeration means, to a site (50, 60) chosen for a one-off or non-permanent use, said transport stage being at least followed by a stage of bringing the temperature of the treatment chamber to its operating temperature.

14. Method according to claim 13, **characterized in that** the transport stage includes a pre-cooling of at least the treatment chamber (13, 53) by the second refrigeration means (412, 512) capable of operating during travel or during stopovers on said travel.

15. Method according to one of claims 13 to 14, **characterized in that** the transport stage is followed by a stage of connecting or securing the treatment chamber (53) to at least one antechamber constructed or transported independently of the treatment chamber.

## Patentansprüche

1. System zur Ganzkörperkryotherapie für menschliche oder tierische Patienten, umfassend wenigstens eine Behandlungskammer (13, 53, 63) mit ausreichenden Abmessungen, um wenigstens einen zu behandelnden Patienten darin aufzunehmen, sowie Kompressor-Kühlmittel (11, 51, 61), die geeignet sind, die Atmosphäre der Behandlungskammer auf eine Temperatur von unter -80 °C zu bringen oder auf dieser zu halten, **dadurch gekennzeichnet, dass** die Behandlungskammer und die Kühlmittel, oder wenigstens eines dieser beiden Elemente, an wenigstens einer mobilen (100, 41, 59, 501) oder transportierbaren (61) selbsttragenden Struktur angebracht sind und an der selbstragenden Struktur angeordnet sind, um eine Durchführung von der selbsttragenden Struktur aus zu ermöglichen.

2. System nach dem vorhergehenden Anspruch, **dadurch gekennzeichnet, dass** die selbsttragende Struktur (100, 41, 501, 61) in ein Straßenfahrzeug (19, 59) oder einen Straßenanhänger (100) integriert ist oder Mittel zum Befestigen an einem solchen Fahrzeug (69) oder Anhänger oder Greifmittel umfasst, die die Handhabung der Struktur durch Hub- oder Fördermittel ermöglichen.

3. System nach einem der vorhergehenden Ansprüche, **dadurch gekennzeichnet, dass** die Kühlmittel (11, 51, 61) über wenigstens eine Hängestruktur, die angeordnet ist, um eine Durchführung von der Hängestruktur aus zu ermöglichen, aufgehängt sind.

4. System nach einem der vorhergehenden Ansprüche, **dadurch gekennzeichnet, dass** die Behandlungskammer (13, 53, 63) durch eine oder mehrere aufeinanderfolgende Vorkammern (14, 15, 54, 55, 64, 65), die jeweils ausreichende Abmessungen aufweisen, um wenigstens einen zu behandelnden Patienten darin aufzunehmen, und deren jeweilige Atmosphären auf eine oder mehrere Zwischentemperaturen zwischen der Außentemperatur und der Temperatur der Behandlungskammer gebracht oder auf dieser gehalten werden, mit der Außenseite in Verbindung steht.

5. System nach Anspruch 4, **dadurch gekennzeichnet, dass** es Mittel zur lokalen Kryotherapie umfasst, die vom Innenraum wenigstens einer Vorkammer aus verwendbar sind.

6. System nach einem der Ansprüche 4 bis 5, **dadurch gekennzeichnet, dass** wenigstens eine Vorkammer (14, 15, 54, 55, 64, 65) eine Vorrichtung zur räumlichen Trennung (142, 152, 642, 652) umfasst, die wenigstens zwei getrennte Strecken zwischen einerseits wenigstens einer Öffnung (131), die mit dem benachbarten Raum (13) mit einer Temperatur unterhalb der Temperatur der Vorkammer (14) in Verbindung steht, und andererseits wenigstens einer Öffnung (141), die mit dem benachbarten Raum (15) mit einer Temperatur oberhalb der Temperatur der Vorkammer in Verbindung steht, definiert.

7. System nach einem der Ansprüche 4 bis 6, **dadurch gekennzeichnet, dass** es eine oder mehrere bewegliche oder abnehmbare Trennwände umfasst, die wenigstens eine Vorrichtung zur räumlichen Trennung (145) zwischen zwei Vorkammern (14 und 15) oder innerhalb (142, 152) einer Vorkammer (14, 15) ausbilden.

8. System nach einem der vorhergehenden Ansprüche, **dadurch gekennzeichnet, dass** es wenigstens zwei trennbare Module umfasst, von denen wenigstens eines an einer mobilen (48; 59) oder transportierbaren (61) selbsttragenden Struktur angebracht ist und an der selbsttragenden Struktur angeordnet ist, um eine Durchführung von der selbsttragenden Struktur aus zu ermöglichen, wobei das eine (41; 501; 60) der Module wenigstens die Behandlungskammer (13; 53; 63) umfasst und das andere (42; 50; 61) der Module wenigstens die Kühlmittel (61) oder wenigstens eine Vorkammer (14, 15; 54, 55) umfasst.

9. System nach einem der Ansprüche 2 bis 8, **dadurch gekennzeichnet, dass** das Straßenfahrzeug oder der Straßenanhänger (100) wenigstens ein bewegliches Teil (101, 103) umfasst, das eine Veränderung der horizontalen Abmessungen von wenigstens einem Element (12, 13, 14, 15), das es trägt, ermöglicht.

10. System nach einem der Ansprüche 8 bis 9, **dadurch gekennzeichnet, dass** die Kühlmittel an einer mobilen oder transportierbaren (61) selbsttragenden Struktur angebracht sind, wobei das System Verbindungsmittel (68) umfasst, um die Kühlmittel (61) mit einer oder mehreren von den Kühlmitteln unabhängig aufgebauten oder transportierten Behandlungskammern (63) lösbar zu verbinden.

11. System nach einem der Ansprüche 8 bis 10, **dadurch gekennzeichnet, dass** die Kühlmittel (51) und die Behandlungskammer (53) an einer oder mehreren mobilen (59, 501) oder transportierbaren selbsttragenden Strukturen angebracht sind, wobei das System Verbindungsmittel (547, 548) umfasst, um die Behandlungskammer (53) mit einer oder mehreren von den Kühlmitteln unabhängig aufgebauten oder transportierten Vorkammern (54, 55) lösbar zu verbinden.

12. System nach einem der vorhergehenden Ansprüche, **dadurch gekennzeichnet, dass** es ferner zweite Kühlmittel (412, 512, 592, 612) umfasst, die den Betrieb von Mitteln zur lokalen Kryotherapie (149, 159, 549, 559, 649, 659) übernehmen oder das vollständige oder teilweise Erreichen oder Temperaturhalten von wenigstens einer Vorkammer (14, 15, 54, 55, 64, 65), einer Temperatur oberhalb der Behandlungskammer übernehmen, oder eine Ergänzung oder eine Vorbereitung für das Erreichen oder Temperaturhalten der Behandlungskammer (13, 53, 63) übernehmen.

13. Verfahren zur Vorbereitung eines Systems nach einem der vorhergehenden Ansprüche, **dadurch gekennzeichnet, dass** es einen Schritt zum Transportieren der Kühlmittel (11, 51, 61) und der Behandlungskammer (13, 53), oder wenigstens der Kühlmittel, an einen für eine punktuelle oder nicht permanente Verwendung gewählten Standort (50, 60) umfasst, wobei sich an den Transportschritt wenigstens ein Schritt zum Temperieren der Behandlungskammer bis auf ihre Betriebstemperatur anschließt.

14. Verfahren nach Anspruch 13, **dadurch gekennzeichnet, dass** der Transportschritt ein Vorkühlen wenigstens der Behandlungskammer (13, 53) durch zweite Kühlmittel (412, 512), die geeignet sind, während der Fahrt oder der Etappen der Fahrt zu arbeiten, einschließt.

15. Verfahren nach einem der Ansprüche 13 bis 14, **dadurch gekennzeichnet, dass** sich an den Transportschritt ein Schritt zum Verbinden oder Befestigen der Behandlungskammer (53) mit/an wenigstens einer von der Behandlungskammer unabhängig aufgebauten oder transportierten Vorkammer (54) anschließt.
